# EUROPÄISCHE PATENTSCHRIFT

(11) **EP 3 920 831 B1**
(45) Veröffentlichungstag und Bekanntmachung des Hinweises auf die Patenterteilung: **26.03.2025**
(21) Anmeldenummer: 20701952.2
(22) Anmeldetag: 21.01.2020
(51) Int. Cl.: A61B 90/70, A61L 2/00

(54) **SYSTEM UND VERFAHREN ZUM STERILISIEREN VON MEDIZINISCHEN INSTRUMENTEN**
SYSTEM AND METHOD FOR STERILIZING MEDICAL INSTRUMENTS
SYSTÈME ET PROCÉDÉ DE STÉRILISATION D'INSTRUMENTS MÉDICAUX

(30) Priorität: 04.02.2019 DE 102019201373
(43) Veröffentlichungstag der Anmeldung: 15.12.2021
(73) Patentinhaber: KUKA Deutschland GmbH, 86165 Augsburg (DE)
(72) Erfinder: KEIBEL, Andreas, 86161 Augsburg (DE)
(74) Vertreter: Oelke, Jochen
(86) Internationale Anmeldenummer: PCT/EP2020/051323
(87) Internationale Veröffentlichungsnummer: WO 2020/160896

(56) Entgegenhaltungen:
- WO-A1-2018/087225
- WO-A1-2018/228818
- US-A1- 2018 280 112

## Beschreibung

Die vorliegende Erfindung betrifft ein System und ein Verfahren zum Sterilisieren von medizinischen Instrumenten.

Wiederverwendbare medizinische Instrumente werden nach interner Praxis typischerweise gereinigt, desinfiziert und anschließend gelagert. Mit diesen gereinigt gelagerten Instrumenten werden Siebe bestückt, die anschließend sterilisiert und dann erneut verwendet werden, wobei der oben beschriebene Zyklus in der Regel mehrfach durchlaufen wird.

Die WO 2018228818 A1 offenbart ein System und Verfahren zur zumindest teilautomatisierten Handhabung eines Instrumentensiebs zur Aufbewahrung und Bereitstellung von chirurgischen Instrumenten.

Aus der WO 2018087225 A1 ist ein Prozess zur Aufarbeitung von chirurgischen Instrumenten bekannt, aufweisend zwischen einem Eingangs- und einem Ausgangsbereich verschiedene Arbeitsstationen wie z.B. Desinfektions- und Bereitstellungs-, sowie Pack- und Lagerbereiche.

Aufgabe der vorliegenden Erfindung ist es, das Sterilisieren medizinischer Instrumente zu verbessern.

Diese Aufgabe wird durch ein System mit den Merkmalen des Anspruchs 1 bzw. ein Verfahren mit den Merkmalen des Anspruchs 7 gelöst. Entsprechend ist in einer Ausführung das System, insbesondere hard- und/oder software-, insbesondere programmtechnisch, zur Durchführung eines hier beschriebenen Verfahrens eingerichtet bzw. wird in einer Ausführung das Verfahren mittels eines hier beschriebenen Systems durchgeführt. Die Unteransprüche betreffen vorteilhafte Weiterbildungen.

Nach einer Ausführung der vorliegenden Erfindung weist ein erfindungsgemäßes System zum Sterilisieren von medizinischen Instrumenten die Merkmale des beiliegenden, unabhängigen Vorrichtungsanspruchs 1 auf.

Nach einer Ausführung der vorliegenden Erfindung weist ein erfindungsgemäßes Verfahren zum Sterilisieren von medizinischen Instrumenten, in einer Ausführung mittels eines hier beschriebenen Systems, die Merkmale des beiliegenden, unabhängigen Verfahrensanspruchs 7 auf.

Durch das robotische Transportieren bzw. Bestücken kann in einer Ausführung das Sterilisieren medizinischer Instrumente vorteilhaft wenigstens teilweise automatisiert und hierdurch dessen Prozesszeit und/oder -zuverlässigkeit verbessert werden, durch das vorhergehende Prüfen und/oder das Zwischenlagern von Instrumenten zwischen dem Prüfen und Bestücken in einer Ausführung das wenigstens teilweise robotische Sterilisieren, insbesondere dessen Variabilität und/oder Zuverlässigkeit, (weiter) verbessert werden.

Die medizinischen Instrumente sind in einer Ausführung Instrumente für, insbesondere klinische, Operationen, insbesondere also chirurgische Instrumente, sie können Operationsbesteck wie Skalpelle, Zangen, Scheren, Nadeln, Sägen und dergleichen aufweisen, insbesondere sein. Die vorliegende Erfindung kann bei solchen Instrumenten, insbesondere aufgrund ihres hohen Durchsatzes und/oder erforderlicher kurzer Prozesszeiten und/oder hoher Prozesssicherheiten beim Sterilisieren, mit besonderem Vorteil verwendet werden.

Die Sterilisiervorrichtung weist in einer Ausführung wenigstens einen, bevorzugt mehrere, Autoklaven auf. Hierdurch kann in einer Ausführung das Sterilisieren verbessert werden. In einer Weiterbildung werden mit Instrumenten bestückte Instrumententräger selektiv einem von wenigstens zwei Autoklaven zugeführt, in diesem sterilisiert, und aus dem einen Autoklav einem nachfolgend näher erläuterten Instrumententräger-mit-Instrumenten-Lager zugeführt und in diesem bis zu einem Abtransport durch ein nachfolgend näher erläutertes Transportsystem gelagert, und aus dem anderen Autoklav direkt dem Transportsystem zugeführt, ohne in dem Instrumententräger-mit-Instrumenten-Lager gelagert zu werden. Hierdurch kann in einer Ausführung die Prozesseffizienz verbessert werden.

Der bzw. einer oder mehrere der Roboter können (jeweils) einen mehr-, insbesondere wenigstens drei-, in einer Ausführung wenigstens sechs-, insbesondere wenigstens siebenachsigen Roboterarm aufweisen, der aufgrund seiner Variabilität zum robotischen Transportieren bzw. Bestücken besonders vorteilhaft ist.

Wenn in einer Ausführung der bzw. einer oder mehrere der Roboter sowohl zum Transportieren von Instrumenten von der Prüfstation zu dem Instrumentenlager als auch zum Bestücken von Instrumententrägern mit Instrumenten von dem Instrumentenlager verwendet werden, kann hierdurch in einer Ausführung das System vorteilhaft kompakter und/oder einfacher ausgebildet sein.

Wenn in einer Ausführung einer oder mehrere der Roboter zum Transportieren von Instrumenten von der Prüfstation zu dem Instrumentenlager und ein oder mehrere andere der Roboter zum Bestücken von Instrumententrägern mit Instrumenten von dem Instrumentenlager verwendet werden, kann hierdurch in einer Ausführung das System vorteilhaft schnell(er) arbeiten.

Ein Instrumententräger kann in einer Ausführung ein(en) Sieb, insbesondere Siebträger, aufweisen, insbesondere sein. Hierdurch kann in einer Ausführung das Sterilisieren des mit Instrumenten bestückten Instrumententrägers verbessert werden.

In einer Ausführung weist die Prüfstation einen Handarbeitsplatz auf, an dem Instrumente durch eine oder mehrere Bedienpersonen geprüft, insbesondere gewartet, in einer Ausführung gereinigt, gegebenenfalls erneut bzw. nachgereinigt, geschmiert und/oder repariert, und/oder - gegebenenfalls - entsorgt werden, insbesondere Einweg-Instrumente und/oder fehlerhafte bzw. nicht reparierte bzw. reparable Instrumente, bzw. der hierzu vorgesehen, insbesondere eingerichtet, ist bzw. verwendet wird.

Durch einen solchen Handarbeitsplatz können in einer Ausführung Instrumente für das robotische Sterilisieren vorteilhaft vorbereitet und/oder vorsortiert, insbesondere hierfür ungeeignete Instrumente aussortiert, werden. Dadurch kann das robotische Sterilisieren, insbesondere dessen Variabilität, Prozesszeit und/oder Zuverlässigkeit, (weiter) verbessert werden. Gerade durch die Kombination eines Handarbeitsplatzes mit einem robotischen Transport bzw. Bestücken von Instrumenten bzw. Instrumententrägern kann eine besonders hohe Variabilität, Prozessgeschwindigkeit und/oder Zuverlässigkeit erreicht werden.

Zusätzlich oder alternativ weist die Prüfstation in einer Ausführung eine Aufnahmestation auf, an der Instrumente mit dem bzw. den Roboter(n) aufgenommen werden bzw. die hierzu vorgesehen, insbesondere eingerichtet, ist bzw. verwendet wird.

Dadurch kann in einer Ausführung das robotische Sterilisieren, insbesondere dessen Variabilität, Prozesszeit und/oder Zuverlässigkeit, (weiter) verbessert werden.

In einer Ausführung weist die Prüfstation eine passive oder aktuierte bzw. aktive Transporteinrichtung auf, mit der bzw. durch die (geprüfte, insbesondere also gegebenenfalls gewartete) Instrumente passiv oder aktuiert bzw. aktiv von dem Handarbeitsplatz zu der Aufnahmestation der Prüfstation transportiert werden bzw. die hierzu vorgesehen, insbesondere eingerichtet, ist bzw. verwendet wird.

Eine passive Transporteinrichtung kann in einer Ausführung eine oder mehrere Rutschen, Rampen oder dergleichen aufweisen bzw. Instrumente gravitationsbedingt transportieren.

Hierdurch kann in einer Ausführung der Bauraum und/oder Energiebedarf des Systems verbessert werden.

Eine aktuierte bzw. aktive Transporteinrichtung kann in einer Ausführung ein oder mehrere Förderbänder, Förderketten oder dergleichen aufweisen bzw. Instrumente mittels eines oder mehrerer, insbesondere elektrischer, pneumatischer und/oder hydraulischer, Antriebe transportieren.

Hierdurch kann in einer Ausführung die Zuverlässigkeit und/oder Taktzeit des Systems verbessert werden.

Zusätzlich oder alternativ weist die Prüfstation in einer Ausführung eine Instrumentenerkennung auf, mit der bzw. durch die Instrumente an der Aufnahmestation erfasst werden bzw. die hierzu vorgesehen, insbesondere eingerichtet, ist bzw. verwendet wird. In einer Weiterbildung werden Instrumente mit dem bzw. den Roboter(n) auf Basis bzw. in Abhängigkeit von der Instrumentenerkennung aufgenommen, in einer Ausführung eine Greifpose des Roboters zum Aufnehmen eines Instruments und/oder eine Identität eines aufzunehmenden Instruments ermittelt, bzw. ist das System, insbesondere der bzw. die Roboter und/oder die Instrumentenerkennung, hierzu vorgesehen, insbesondere eingerichtet, bzw. wird hierzu verwendet. Zusätzlich oder alternativ werden in einer Weiterbildung Instrumente auf Basis bzw. in Abhängigkeit von der Instrumentenerkennung kontrolliert, insbesondere die Anzahl ihrer Verwendungen bzw. (Sterilisierungs(system))Durchläufe, so dass in einer Ausführung Instrumente auf Basis bzw. in Abhängigkeit von dieser Kontrolle, insbesondere bei Feststellen eines Erreichens einer vorgegebenen maximalen Zyklus- bzw. Verwendungsanzahl, aussortiert bzw. ausgesondert werden, bzw. ist das System, insbesondere der bzw. die Roboter und/oder die Instrumentenerkennung, hierzu vorgesehen, insbesondere eingerichtet, bzw. wird hierzu verwendet.

Zusätzlich oder alternativ weist der Handarbeitsplatz in einer Ausführung eine Reinigungsstation auf, an der Instrumente durch die Bedienperson(en) manuell gereinigt werden, bzw. die hierzu vorgesehen, insbesondere eingerichtet, ist bzw. verwendet wird, in einer Ausführung Waschbehälter mit Reinigungsflüssigkeit und/oder Reinigungswerkzeug aufweist.

Zusätzlich oder alternativ weist der Handarbeitsplatz in einer Ausführung eine Reparaturstation auf, an der Instrumente durch die Bedienperson(en) manuell repariert werden, bzw. die hierzu vorgesehen, insbesondere eingerichtet, ist bzw. verwendet wird, in einer Ausführung Ersatzteile und/oder Reparaturwerkzeug aufweist.

Zusätzlich oder alternativ weist der Handarbeitsplatz in einer Ausführung eine Entsorgungsstation auf, an der Instrumente, insbesondere Einweg-Instrumente und/oder fehlerhafte bzw. nicht reparierte bzw. reparable Instrumente, durch die Bedienperson(en) manuell entsorgt werden, bzw. die hierzu vorgesehen, insbesondere eingerichtet, ist bzw. verwendet wird, in einer Ausführung wenigstens einen Abfall- bzw. Entsorgungsbehälter aufweist, der geleert oder gewechselt wird bzw. hierzu vorgesehen, insbesondere eingerichtet, ist bzw. verwendet wird.

Durch eines und insbesondere eine Kombination mehrerer der oben genannten Merkmale können in einer Ausführung Instrumente für das robotische Sterilisieren vorteilhaft vorbereitet und/oder vorsortiert werden. Dadurch kann das robotische Sterilisieren, insbesondere dessen Variabilität, Prozesszeit und/oder Zuverlässigkeit, (weiter) verbessert werden.

In einer Ausführung weist das Instrumentenlager einen Eingabebereich, insbesondere ein oder mehrere, in einer Ausführung aktuierte bzw. durch Antriebe bewegliche bzw. verstell-, insbesondere öffen- bzw. schließ- und/oder ein- bzw. ausfahrbare, Eingabeflächen, -fächer oder dergleichen auf, an, insbesondere auf bzw. in, denen Instrumente mit dem bzw. den Robotern abgegeben werden, bzw. ist das System, insbesondere der bzw. die Roboter und/oder das Instrumentenlager bzw. dessen Eingabebereich, hierzu vorgesehen, insbesondere eingerichtet, bzw. wird hierzu verwendet.

Zusätzlich oder alternativ weist in einer Ausführung das Instrumentenlager einen, in einer Ausführung von dem Eingabebereich beabstandeten, Ausgabebereich, insbesondere ein oder mehrere, in einer Ausführung aktuierte bzw. durch Antriebe bewegliche bzw. verstell-, insbesondere öffen- bzw. schließ- und/oder ein- bzw. ausfahrbare, Ausgabeflächen, -fächer oder dergleichen auf, an, insbesondere auf bzw. in, denen selektiv, insbesondere auf Basis einer Instrumentenanforderung, die ein individuelles Instrument oder einen Instrumententyp spezifiziert, unterschiedliche Instrumente ausgegeben werden, bzw. ist das Instrumentenlager bzw. dessen Ausgabebereich hierzu vorgesehen, insbesondere eingerichtet, bzw. wird hierzu verwendet. In einer Weiterbildung können auf derselben Ausgabefläche selektiv unterschiedliche Instrumenten ausgegeben werden, was insbesondere den Zugriff durch den bzw. die Roboter verbessern kann.

Zusätzlich oder alternativ weist in einer Ausführung das Instrumentenlager ein, insbesondere aktuiertes Lagersystem auf, durch das ein oder mehrere Instrumente (jeweils), in einer Ausführung auf Basis einer Lagerverwaltung, insbesondere durch diese kommandiert bzw. koordiniert, an einer von mehreren unterschiedlichen (potentiellen bzw. hierzu vorgesehenen, insbesondere eingerichteten, bzw. verwendeten) Lagerstellen abgelegt werden, bzw. ist das Instrumentenlager bzw. dessen Lagersystem hierzu vorgesehen, insbesondere eingerichtet, bzw. wird hierzu verwendet.

Zusätzlich oder alternativ weist in einer Ausführung das Instrumentenlager eine Instrumentenerkennung auf, mit der bzw. durch die Instrumente an dem Ausgabebereich erfasst werden bzw. die hierzu vorgesehen, insbesondere eingerichtet, ist bzw. verwendet wird. In einer Weiterbildung werden Instrumente mit dem bzw. den Roboter(n) auf Basis bzw. in Abhängigkeit von der Instrumentenerkennung aufgenommen, in einer Ausführung eine Greifpose des Roboters zum Aufnehmen eines Instruments und/oder eine Identität eines aufzunehmenden Instruments ermittelt, bzw. ist das System, insbesondere der bzw. die Roboter und/oder die Instrumentenerkennung, hierzu vorgesehen, insbesondere eingerichtet, bzw. wird hierzu verwendet. Zusätzlich oder alternativ werden in einer Weiterbildung Instrumente auf Basis bzw. in Abhängigkeit von der Instrumentenerkennung kontrolliert, insbesondere ihre Identität überprüft, bzw. ist das System, insbesondere die Instrumentenerkennung, hierzu vorgesehen, insbesondere eingerichtet, bzw. wird hierzu verwendet.

In einer Ausführung werden Instrumente in dem Instrumentenlager einzeln gelagert bzw. ist das Instrumentenlager, insbesondere sein Lagersystem bzw. seine Lagerverwaltung, hierzu vorgesehen, insbesondere eingerichtet, bzw. wird hierzu verwendet.

Hierdurch können in einer Ausführung jeweils, insbesondere in Kombination, Instrumente beim robotischen Sterilisieren vorteilhaft zwischengelagert werden. Dadurch kann das robotische Sterilisieren, insbesondere dessen Variabilität, Prozesszeit und/oder Zuverlässigkeit, (weiter) verbessert werden.

In einer Ausführung weist die Packstation eine Instrumentenerkennung auf, mit der bzw. durch die Instrumente und/oder Instrumententräger an der Packstation erfasst werden bzw. die hierzu vorgesehen, insbesondere eingerichtet, ist bzw. verwendet wird. Zur kompakteren Darstellung wird auch eine (reine) Instrumententrägererkennung verallgemeinernd als eine Instrumentenerkennung im Sinne der vorliegenden Erfindung bezeichnet, so dass eine Instrumentenerkennung, insbesondere der Packstation, eine Instrumententräger- und/oder Instrumentenerkennung bzw. eine Instrumenten(träger)erkennung sein kann, mit der bzw. durch die Instrumente und/oder Instrumententräger, insbesondere an der Packstation, erfasst werden bzw. die hierzu vorgesehen, insbesondere eingerichtet, ist bzw. verwendet wird.

In einer Weiterbildung werden Instrumententräger mit dem bzw. den Roboter(n) auf Basis bzw. in Abhängigkeit von der Instrumenten(träger)erkennung mit Instrumenten bestückt, in einer Ausführung eine Ablagepose des Roboters zum Bestücken eines Instrumententrägers mit einem Instrument und/oder eine Lage und/oder Identität eines zu bestückenden Instrumententrägers ermittelt, bzw. ist das System, insbesondere der bzw. die Roboter und/oder die Instrumenten(träger)erkennung, hierzu vorgesehen, insbesondere eingerichtet, bzw. wird hierzu verwendet. Insbesondere können auf Basis mittels der Instrumenten(träger)erkennung identifizierter Instrumententräger hierfür vorgegebene, insbesondere abgespeicherte, Ablagepositionen der Instrumente zum Bestücken vorgegeben werden bzw. sein.

Zusätzlich oder alternativ werden in einer Weiterbildung Instrumententräger und/oder Instrumente, insbesondere Instrumente, mit denen ein Instrumententräger bestückt ist, auf Basis bzw. in Abhängigkeit von der Instrumenten(träger)erkennung kontrolliert, insbesondere ihre Identität und/oder korrekte Bestückung überprüft, bzw. ist das System, insbesondere die Instrumentenerkennung, hierzu vorgesehen, insbesondere eingerichtet, bzw. wird hierzu verwendet.

Hierdurch kann in einer Ausführung die Zuverlässigkeit und/oder Taktzeit des Systems (weiter) verbessert werden.

In einer Ausführung weisen eine oder mehrere der vorstehend genannten Instrumentenerkennungen (jeweils) eine oder mehrere, insbesondere berührungslose, Sende-Empfangs-Vorrichtungen, insbesondere für elektromagnetische Strahlung und/oder zur Identifikation, in einer Ausführung RFID-Sende-Empfangs-Vorrichtungen oder dergleichen, und/oder eine oder mehrere Kameras, in einer Ausführung Stereokameras, insbesondere eine, in einer Ausführung dreidimensionale, Bildverarbeitung auf, mithilfe der (jeweils) eine Lage, insbesondere ein-, zwei- oder dreidimensionale Position und/oder ein-, zwei- oder dreidimensionale Orientierung, und/oder eine Identität des (jeweiligen) Instruments ermittelt werden bzw. die hierzu vorgesehen, insbesondere eingerichtet, sind bzw. verwendet werden. In einer Weiterbildung weisen die Instrumente, insbesondere passive, Sender, in einer Ausführung RFID-Tags oder dergleichen, und/oder, in einer Ausführung zwei- oder dreidimensionale und/oder digitale, Markierungen, insbesondere DataMatrix Codes, Farbkodes, QR-Codes oder dergleichen, auf, die durch die Instrumentenerkennungen erfasst bzw. identifiziert werden.

Hierdurch können in einer Ausführung die Lagen und/oder Identitäten der Instrumente vorteilhaft, insbesondere schnell, präzise und/oder zuverlässig, ermittelt werden.

In einer Ausführung weist das System eine Reinigungs- und/oder Desinfektionsstation, an, insbesondere mittels bzw. durch, der bzw. die Instrumente gereinigt und/oder desinfiziert werden, bzw. die hierzu vorgesehen, insbesondere eingerichtet, ist bzw. verwendet wird, und ein passive oder aktuierte bzw. aktive Anliefereinrichtung auf, mit der bzw. durch die Instrumente passiv oder aktuiert bzw. aktiv von der Reinigungs- und/oder Desinfektionsstation zu der Prüfstation angeliefert werden, bzw. die hierzu vorgesehen, insbesondere eingerichtet, ist bzw. verwendet wird.

Eine passive Anliefereinrichtung kann in einer Ausführung eine oder mehrere Rutschen, Rampen oder dergleichen aufweisen bzw. Instrumente gravitationsbedingt anliefern.

Hierdurch kann in einer Ausführung der Bauraum und/oder Energiebedarf des Systems verbessert werden.

Eine aktuierte bzw. aktive Anliefereinrichtung kann in einer Ausführung ein oder mehrere Förderbänder, Förderketten oder dergleichen aufweisen bzw. Instrumente mittels eines oder mehrerer, insbesondere elektrischer, pneumatischer und/oder hydraulischer, Antriebe transportieren.

Hierdurch kann in einer Ausführung die Zuverlässigkeit und/oder Taktzeit des Systems verbessert werden.

Durch die vorhergehende Reinigung bzw. Desinfektion kann in einer Ausführung vorteilhaft Bedienpersonal an der Prüfstation geschützt bzw. entlastet werden.

In einer Ausführung weist das System ein Instrumententräger-mit-Instrumenten-Lager auf, durch das, insbesondere in dem, mit Instrumenten bestückte Instrumententräger von der Sterilisiervorrichtung gelagert werden, bzw. das hierzu vorgesehen, insbesondere eingerichtet, ist bzw. verwendet wird.

Hierdurch können in einer Ausführung vorteilhaft bereits sterilisierte, mit (sterilisierten) Instrumenten bestückte Instrumententrägern gelagert und so bei Bedarf schnell(er) ausgeliefert werden.

Zusätzlich oder alternativ weist das System in einer Ausführung ein Instrumententräger-ohne-Instrumente-Lager auf, durch das, insbesondere in dem, leere Instrumententräger, insbesondere an der Reinigungs- und/oder Desinfektionsstation bzw. vor der Prüfstation geleerte Instrumententräger, gelagert werden, bzw. das hierzu vorgesehen, insbesondere eingerichtet, ist bzw. verwendet wird.

Zusätzlich oder alternativ weist das System in einer Ausführung ein Transportsystem auf, mit dem, insbesondere durch das, leere Instrumententräger von der Reinigungs- und/oder Desinfektionsstation und/oder der Prüfstation und/oder dem Instrumententräger-ohne-Instrumente-Lager und/oder zu dem Instrumententräger-ohne-Instrumente-Lager und/oder der Packstation (ab)transportiert werden, bzw. das hierzu vorgesehen, insbesondere eingerichtet, ist bzw. verwendet wird.

Hierdurch können in einer Ausführung, jeweils, insbesondere in Kombination, Instrumententräger, mit denen Instrumente an die Reinigungs- und/oder Desinfektionsstation bzw. Prüfstation angeliefert werden, vorteilhaft wiederverwendet werden.

Zusätzlich oder alternativ weist das System in einer Ausführung ein Transportsystem auf, mit dem, insbesondere durch das, mit Instrumenten bestückte Instrumententräger von der Packstation und/oder der Sterilisiervorrichtung und/oder dem Instrumententräger-mit-Instrumenten-Lager und/oder zu der Sterilisiervorrichtung und/oder dem Instrumententräger-mit-Instrumenten-Lager (ab)transportiert werden, bzw. das hierzu vorgesehen, insbesondere eingerichtet, ist bzw. verwendet wird, in einer Weiterbildung ein Transportsystem mit fahrerlosen Transportfahrzeugen, die mit Instrumenten bestückte Instrumententräger von der Sterilisiervorrichtung und/oder dem Instrumententräger-mit-Instrumenten-Lager (ab)transportieren bzw. hierzu vorgesehen, insbesondere eingerichtet, sind bzw. verwendet werden.

Das Transportsystem weist in einer Ausführung eine oder mehrere passive Transporteinrichtungen, insbesondere eine oder mehrere Rutschen, Rampen oder dergleichen auf. Hierdurch kann in einer Ausführung der Bauraum und/oder Energiebedarf des Systems verbessert werden.

Zusätzlich oder alternativ weist das Transportsystem in einer Ausführung eine oder mehrere aktuierte bzw. aktive Transporteinrichtungen, insbesondere ein oder mehrere Förderbänder, Förderketten oder dergleichen auf. Hierdurch kann in einer Ausführung die Zuverlässigkeit und/oder Taktzeit des Systems verbessert werden.

In einer Ausführung werden ein oder mehrere, insbesondere alle, Schritte des Verfahrens vollständig oder teilweise automatisiert durchgeführt, insbesondere durch das System bzw. sein(e) Mittel. In einer Ausführung weist das System die Instrumente auf.

Weitere Vorteile und Merkmale ergeben sich aus den Unteransprüchen und den Ausführungsbeispielen. Hierzu zeigt, teilweise schematisiert, die einzige:

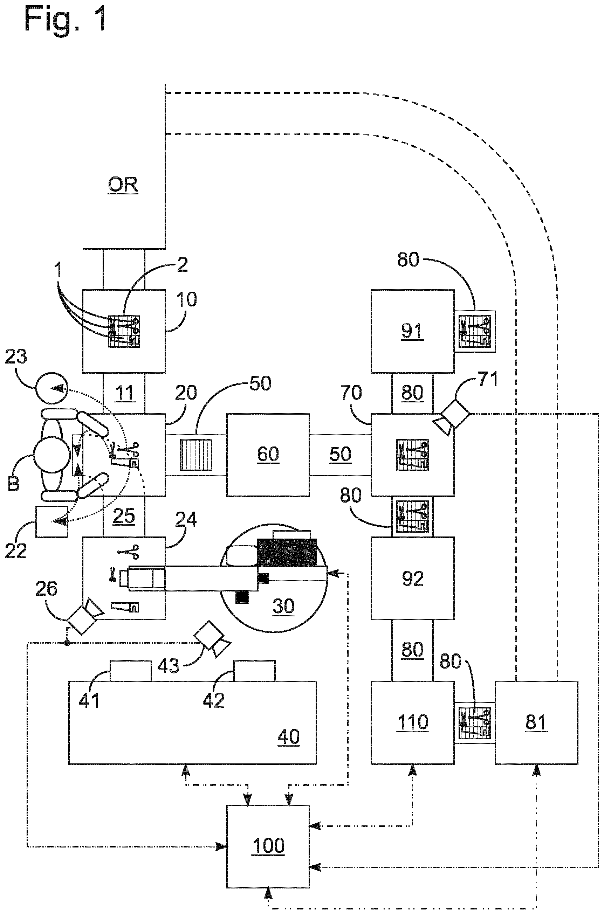

- Fig. 1:: ein Verfahren und System zum Sterilisieren von medizinischen Instrumenten nach einer Ausführung der vorliegenden Erfindung.

Fig. 1 zeigt ein System und zugleich auch ein Verfahren zum Sterilisieren von medizinischen Instrumenten nach einer Ausführung der vorliegenden Erfindung.

Das System weist eine Reinigungs- und Desinfektionsstation 10 auf, in der gebrauchte klinische OP-Instrumente 1 aus einem Operationsraum OR in Siebträgern 2 gereinigt und desinfiziert werden.

Eine Anliefereinrichtung, beispielsweise ein Förderband 11, liefert diese Instrumente in den Siebträgern 2 von der Reinigungs- und Desinfektionsstation an eine Prüfstation.

Die Prüfstation weist einen Handarbeitsplatz mit einem Arbeitstisch 20, an dem eine Bedienperson B Instrumente manuell prüft und gegebenenfalls repariert (Reparaturstation), eine Reinigungsstation, im Ausführungsbeispiel ein Waschbecken 22, an der die Bedienperson B Instrumente gegebenenfalls manuell nachreinigt und eine Entsorgungsstation mit einem Abfallbehälter 23, in den die Bedienperson B nicht reparable Instrumente, falsch angelieferte Einweginstrumente und dergleichen manuell entsorgt, sowie eine Aufnahmestation in Form einer Erkennungsplattform 24 zum Aufnehmen von Instrumenten mit einem Roboter 30 auf.

Die Prüfstation weist weiter eine passive oder aktuierte Transporteinrichtung 25, beispielsweise eine Rutsche oder ein Förderband, auf, mit der Instrumente von dem Handarbeitsplatz 20 zu der Aufnahmestation 24 transportiert werden. Ergonomisch vorteilhaft weist die Transporteinrichtung einen Eingabeschacht am Arbeitstisch 20 auf und ist unter dem Arbeitstisch 20 hindurchgeführt, in Fig. 1 exemplarisch in Form einer gestrichelten Transportbahn angedeutet. Das Reinigen, Entsorgen bzw. Einwerfen in den Eingabeschacht ist in Fig. 1 durch gepunktete Pfeile angedeutet.

Eine Instrumentenerkennung 26 mit einer Kamera mit Bilderkennung erfasst Instrumente an der Aufnahmestation 24. Zusätzlich oder alternativ kann auch eine RFID-Sende-Empfangs-Vorrichtung verwendet werden, die ebenfalls durch die Instrumentenerkennung 26 symbolisiert sein soll. Diese kann in ein Instrumenten-Lebenszyklusmanagement (Instrument PLM) eingebunden sein, das überwacht, wie oft ein individuelles Instrument bereits das System durchlaufen hat, und gegebenenfalls ein Aussortieren bei Erreichen einer vorgegebenen Maximalzahl kommandiert. Gleiches kann auch durch eine Erkennung von DatenMatrix-Codes oder dergleichen an den Instrumenten realisiert werden.

Der Roboter nimmt Instrumente von der Aufnahmestation 24 auf. Dabei kann mittels der Instrumentenerkennung 26 insbesondere eine Greifpose des Roboters ermittelt werden, insbesondere auf Basis einer durch die Instrumentenerkennung 26 erfassten Lage der Instrumente. In einer Ausführung kann auf Basis der Identifizierung individueller Instrumente, beispielsweise auf Basis ihrer RFID-Tags, DatenMatrix-Codes oder dergleichen, eine instrumentenspezifisch abgespeicherte Greifposition am Instrument angefahren werden.

Der Roboter 30 transportiert Instrumente von der Aufnahmestation 24 der Prüfstation zu einem Eingabebereich 41 eines Instrumentenlagers 40, in dem diese Instrumente mittels eines Lagersystems (nicht dargestellt) zwischengelagert werden.

Die an der Prüfstation geleerten Siebträger 2 werden durch ein Transportsystem 50 in ein Instrumententräger-ohne-Instrumente-Lager 60 abtransportiert und in diesem gelagert. Das Transportsystem 50 transportiert leere Siebträger 2 aus dem Instrumententräger-ohne-Instrumente-Lager 60 zu einer Packstation 70. In einer nicht dargestellten Abwandlung können die Siebträger 2 auch in der Reinigungs- und Desinfektionsstation 10 geleert und von dieser durch das Transportsystem 50 zu dem Instrumententräger-ohne-Instrumente-Lager 60 abtransportiert, in diesem gelagert und von dort durch das Transportsystem 50 zu der Packstation 70 transportiert werden.

Der Roboter 30 oder in einer nicht dargestellten Abwandlung ein weiterer Roboter entnimmt einem Ausgabebereich 42 des Instrumentenlagers 40, an dem dessen Lagersystem selektiv Instrumente ausgibt, diese Instrumente und bestückt damit die an der Packstation 70 bereitgestellten Siebträger.

Dabei fordert eine Steuerung 100 auf Basis operationsspezifisch vorgegebener Packmuster für die Bestückung der Siebträger mit Instrumenten, die beispielsweise von den jeweiligen Fachärzten bzw. Operateuren definiert werden, die entsprechenden Instrumente vom Instrumentenlager 40 bzw. an dessen Ausgabebereich 42 an und steuert den Roboter auf Basis des jeweiligen operationsspezifisch vorgegebenen Packmusters zum Bestücken des jeweiligen Siebträgers.

Das Instrumentenlager 40 weist eine Instrumentenerkennung 43 mit einer Kamera mit Bilderkennung auf, die Instrumente an dem Ausgabebereich 42 erfasst. Zusätzlich oder alternativ kann auch eine RFID-Sende-Empfangs-Vorrichtung verwendet werden, die ebenfalls durch die Instrumentenerkennung 43 symbolisiert sein soll.

Der Roboter nimmt Instrumente von dem Ausgabebereich 42 auf. Dabei kann mittels der Instrumentenerkennung 43 insbesondere eine Greifpose des Roboters ermittelt werden, insbesondere auf Basis einer durch die Instrumentenerkennung 43 erfassten Lage des jeweiligen Instrumentes. In einer Ausführung kann auf Basis der Identifizierung individueller Instrumente, beispielsweise auf Basis ihrer RFID-Tags, DatenMatrix-Codes oder dergleichen, eine instrumentenspezifisch abgespeicherte Greifposition am Instrument angefahren werden.

Auch die Packstation 70 weist eine Instrumenten(träger)erkennung 71 mit einer Kamera mit Bilderkennung auf, die Siebträger und Instrumente an der Packstation 70 erfasst.

Der Roboter bestückt die Siebträger mit den Instrumenten von dem Ausgabebereich 42. Dabei kann mittels der Instrumenten(träger)erkennung 71 insbesondere eine Ablagepose des Roboters ermittelt werden, insbesondere auf Basis einer durch die Instrumenten(träger)erkennung 71 erfassten Lage des jeweiligen Siebträgers. In einer Ausführung kann auf Basis einer Identifizierung individueller Siebträger, beispielsweise auf Basis ihrer RFID-Tags, DatenMatrix-Codes oder dergleichen, eine sieb- bzw. instrumententrägerspezifisch abgespeicherte Greifposition am Siebträger angefahren werden.

Abschließend kann die Instrumenten(träger)erkennung 71 die Bestückung des Siebträgers mit den Instrumenten kontrollieren.

Ein Transportsystem 80 transportiert die fertig bestückten Siebträger selektiv zu einem von zwei Autoklaven 91, 92 in denen diese sterilisiert werden.

Anschließend transportiert das Transportsystem 80 die sterilisierten Siebträger aus dem einen Autoklav 92 in ein Instrumententräger-mit-Instrumenten-Lager 110, in dem diese gelagert werden, und aus diesem oder direkt von dem anderen Autoklav 91 zu fahrerlosen Transportfahrzeugen 81 des Transportsystems, die die bestückten, sterilisierten Siebträger zu dem Operationsraum OR befördern, wie in Fig. 1 exemplarisch in Form einer gestrichelten Transportbahn angedeutet.

Obwohl in der vorhergehenden Beschreibung exemplarische Ausführungen erläutert wurden, sei darauf hingewiesen, dass eine Vielzahl von Abwandlungen möglich ist. Außerdem sei darauf hingewiesen, dass es sich bei den exemplarischen Ausführungen lediglich um Beispiele handelt, die den Schutzbereich, die Anwendungen und den Aufbau in keiner Weise einschränken sollen. Vielmehr wird dem Fachmann durch die vorausgehende Beschreibung ein Leitfaden für die Umsetzung von mindestens einer exemplarischen Ausführung gegeben, wobei diverse Änderungen, insbesondere in Hinblick auf die Funktion und Anordnung der beschriebenen Bestandteile, vorgenommen werden können, ohne den Schutzbereich zu verlassen, wie er sich aus den Ansprüchen und diesen äquivalenten Merkmalskombinationen ergibt.

### Bezugszeichenliste

- 1: Instrument(e)
- 2: Siebträger (Instrumententräger)
- 10: Reinigungs- und/oder Desinfektionsstation
- 11: Anliefereinrichtung
- 20: Arbeitstisch (Handarbeitsplatz; Prüf- bzw. Reparaturstation)
- 22: Waschbecken (Reinigungsstation)
- 23: Abfallbehälter (Entsorgungsstation)
- 24: Aufnahmestation
- 25: Transporteinrichtung
- 26: Instrumentenerkennung
- 30: Roboter
- 40: Instrumentenlager
- 41: Eingabebereich
- 42: Ausgabebereich
- 43: Instrumentenerkennung
- 50: Transportsystem
- 60: Instrumententräger-ohne-Instrumente-Lager
- 70: Packstation
- 71: Instrumenten(träger)erkennung
- 80: Transportsystem
- 81: fahrerloses Transportfahrzeug
- 91, 92: Autoklav (Sterilisiervorrichtung)
- 100: Steuerung
- 110: Instrumententräger-mit-Instrumenten-Lager

- B: Bedienperson
- OR: Operationsraum

## Patentansprüche

1. System zum Sterilisieren von medizinischen Instrumenten (1), das aufweist:
- eine Prüfstation (20-26) zum Prüfen angelieferter Instrumente;
- ein Instrumentenlager (40) zum Zwischenlagern geprüfter Instrumente;
- eine Packstation (70) zum Bestücken von Instrumententrägern (2) mit Instrumenten aus dem Instrumentenlager;
- wenigstens einen Roboter (30) zum Transportieren von Instrumenten von der Prüfstation zu dem Instrumentenlager und/oder zum Bestücken von Instrumententrägern an der Packstation mit Instrumenten von dem Instrumentenlager, insbesondere auf Basis eines operationsspezifisch vorgegebenen Packmusters; und
- eine Sterilisiervorrichtung (91, 92) zum Sterilisieren von mit Instrumenten bestückten Instrumententrägern von der Packstation
**dadurch gekennzeichnet, dass** das Instrumentenlager aufweist:
- einen Eingabebereich (41) und einen, insbesondere hiervon beabstandeten, Ausgabebereich (42) zum selektiven Ausgeben unterschiedlicher Instrumente;
- ein Lagersystem zum Ablegen eines Instrumentes an unterschiedlichen Lagerstellen; und
- eine Instrumentenerkennung (43) zum Erfassen von Instrumenten an dem Ausgabebereich, insbesondere zum Aufnehmen von Instrumenten mit dem wenigstens einen Roboter und/oder Kontrollieren der Instrumente.

2. System nach Anspruch 1, **dadurch gekennzeichnet, dass** die Prüfstation aufweist:
- einen Handarbeitsplatz (20-23) zum Prüfen, insbesondere Warten und/oder Entsorgen, von Instrumenten durch wenigstens eine Bedienperson; und/oder
- eine Aufnahmestation (24) zum Aufnehmen von Instrumenten mit dem wenigstens einen Roboter.

3. System nach dem vorhergehenden Anspruch, **dadurch gekennzeichnet, dass**
- die Prüfstation:
- eine passive oder aktuierte Transporteinrichtung (25) zum Transportieren von Instrumenten von dem Handarbeitsplatz zu der Aufnahmestation; und/oder
- eine Instrumentenerkennung (26) zum Erfassen von Instrumenten an der Aufnahmestation, insbesondere zum Aufnehmen von Instrumenten mit dem wenigstens einen Roboter und/oder Kontrollieren der Instrumente; und/oder
- der Handarbeitsplatz
- eine Reinigungsstation (22) zum manuellen Reinigen von Instrumenten;
- eine Reparaturstation (20) zum manuellen Reparieren von Instrumenten; und/oder
- eine Entsorgungsstation (23) zum manuellen Entsorgen von Instrumenten aufweist.

4. System nach einem der vorhergehenden Ansprüche, **dadurch gekennzeichnet, dass** die Packstation aufweist:
- eine Instrumentenerkennung (71) zum Erfassen von Instrumenten und/oder Instrumententrägern an der Packstation, insbesondere zum Bestücken von Instrumententrägern mit Instrumenten mit dem wenigstens einen Roboter und/oder Kontrollieren der Instrumententräger und/oder Instrumente.

5. System nach einem der vorhergehenden Ansprüche, **dadurch gekennzeichnet, dass** die Instrumentenerkennung (26) zum Erfassen von Instrumenten an der Aufnahmestation, die Instrumentenerkennung (43) zum Erfassen von Instrumenten an dem Ausgabebereich und/oder die Instrumentenerkennung (71) zum Erfassen von Instrumenten an der Packstation wenigstens eine Sende-Empfangs-Vorrichtung und/oder wenigstens eine Kamera, insbesondere eine Bildverarbeitung, zum Ermitteln einer Lage und/oder Identität der Instrumente aufweist.

6. System nach einem der vorhergehenden Ansprüche, **gekennzeichnet durch**:
- eine Reinigungs- und/oder Desinfektionsstation (10) zum Reinigen und/oder Desinfizieren von Instrumenten und ein passive oder aktuierte Anliefereinrichtung (11) zum Anliefern dieser Instrumente von der Reinigungs- und/oder Desinfektionsstation zu der Prüfstation; und/oder **durch**:
- ein Instrumententräger-mit-Instrumenten-Lager (110) zum Lagern von mit Instrumenten bestückten Instrumententrägern von der Sterilisiervorrichtung;
- ein Instrumententräger-ohne-Instrumente-Lager (60) zum Lagern von leeren Instrumententrägern; und/oder
- ein Transportsystem (50; 80) zum Abtransportieren von leeren Instrumententrägern von der Reinigungs- und/oder Desinfektionsstation und/oder der Prüfstation und/oder dem Instrumententräger-ohne-Instrumente-Lager und/oder zu dem Instrumententräger-ohne-Instrumente-Lager und/oder der Packstation und/oder Abtransportieren von mit Instrumenten bestückten Instrumententrägern von der Packstation und/oder der Sterilisiervorrichtung und/oder dem Instrumententräger-mit-Instrumenten-Lager und/oder zu der Sterilisiervorrichtung und/oder dem Instrumententräger-mit-Instrumenten-Lager, insbesondere mithilfe fahrerloser Transportfahrzeuge (81).

7. Verfahren zum Sterilisieren von medizinischen Instrumenten (1) mittels eines Systems nach einem der vorhergehenden Ansprüche, mit den Schritten:
- Prüfen angelieferter Instrumente an einer bzw. der Prüfstation (20-26);
- Zwischenlagern geprüfter Instrumente mittels eines bzw. des Instrumentenlagers (40);
- Sterilisieren von mit Instrumenten bestückten Instrumententrägern (2) von einer bzw. der Packstation an einer bzw. der Sterilisiervorrichtung (91, 92); und wenigstens einem der Schritte:
- Transportieren von Instrumenten von der Prüfstation zu dem Instrumentenlager und/oder Bestücken von Instrumententrägern an der Packstation mit Instrumenten von dem Instrumentenlager mit wenigstens einem bzw. dem wenigstens einen Roboter (30), insbesondere auf Basis eines operationsspezifisch vorgegebenen Packmusters.

8. Verfahren nach dem vorhergehenden Anspruch, **gekennzeichnet durch** wenigstens einen der Schritte:
- Prüfen, insbesondere Warten und/oder Entsorgen, von Instrumenten **durch** wenigstens eine Bedienperson (B) an einem bzw. dem Handarbeitsplatz (20-23); und/oder
- Aufnehmen von Instrumenten mit dem wenigstens einen Roboter an einer bzw. der Aufnahmestation (24) der Prüfstation.

9. Verfahren nach dem vorhergehenden Anspruch, **gekennzeichnet durch** wenigstens einen der Schritte:
- passives oder aktuiertes Transportieren von Instrumenten von dem Handarbeitsplatz zu der Aufnahmestation der Prüfstation mit einer bzw. der Transporteinrichtung (25);
- Erfassen von Instrumenten an der Aufnahmestation mit einer bzw. der Instrumentenerkennung (26), insbesondere Aufnehmen von Instrumenten mit dem wenigstens einen Roboter und/oder Kontrollieren der Instrumente auf Basis der Instrumentenerkennung;
- manuelles Reinigen von Instrumenten an einer bzw. der Reinigungsstation (22);
- manuelles Reparieren von Instrumenten an einer bzw. der Reparaturstation (20); und/oder
- manuelles Entsorgen von Instrumenten an einer bzw. der Entsorgungsstation (23).

10. Verfahren nach einem der vorhergehenden Ansprüche 7 bis 9, **gekennzeichnet durch** wenigstens einen der Schritte:
- Abgeben von Instrumenten mit dem wenigstens einen Roboter an einem bzw. dem Eingabebereich (41) des Instrumentenlagers;
- selektiven Ausgeben unterschiedlicher Instrumente an einem bzw. dem Ausgabebereich (42) des Instrumentenlagers;
- Ablegen wenigstens eines Instrumentes an einer von mehreren unterschiedlichen Lagerstellen **durch** ein bzw. das Lagersystem auf Basis einer Lagerverwaltung; und/oder
- Erfassen von Instrumenten an einem bzw. dem Ausgabebereich (42) mit einer bzw. der Instrumentenerkennung (43), insbesondere Aufnehmen von Instrumenten mit dem wenigstens einen Roboter und/oder Kontrollieren der Instrumente auf Basis der Instrumentenerkennung.

11. Verfahren nach einem der vorhergehenden Ansprüche 7 bis 10, **gekennzeichnet durch** den Schritt:
- Erfassen von Instrumenten und/oder Instrumententräger an der Packstation, insbesondere Bestücken von Instrumententrägern mit Instrumenten mit dem wenigstens einen Roboter und/oder Kontrollieren der Instrumententräger und/oder Instrumente, auf Basis einer bzw. der Instrumentenerkennung (71).

12. Verfahren nach einem der vorhergehenden Ansprüche 7 bis 11, **gekennzeichnet durch** wenigstens einen der Schritte:
- Reinigen und/oder Desinfizieren von Instrumenten an einer bzw. der Reinigungs- und/oder Desinfektionsstation (10) und passives oder aktuiertes Anliefern dieser Instrumente von der Reinigungs- und/oder Desinfektionsstation zu der Prüfstation mit einer bzw. der Anliefereinrichtung (11);
- Lagern von mit Instrumenten bestückten Instrumententrägern von der Sterilisiervorrichtung in einem bzw. dem Instrumententräger-mit-Instrumenten-Lager (110);
- Lagern von leeren Instrumententrägern in einem bzw. dem Instrumententräger-ohne-Instrumente-Lager (60); und/oder
- Abtransportieren von leeren Instrumententrägern von einer bzw. der Reinigungs- und/oder Desinfektionsstation (10) und/oder der Prüfstation und/oder einem bzw. dem Instrumententräger-ohne-Instrumente-Lager (60) und/oder zu einem bzw. dem Instrumententräger-ohne-Instrumente-Lager (60) und/oder der Packstation und/oder Abtransportieren von mit Instrumenten bestückten Instrumententrägern von der Packstation und/oder der Sterilisiervorrichtung und/oder dem Instrumententräger-mit-Instrumenten-Lager und/oder zu der Sterilisiervorrichtung und/oder dem Instrumententräger-mit-Instrumenten-Lager mit einem bzw. dem Transportsystem (50; 80), insbesondere mithilfe seiner fahrerlosen Transportfahrzeuge (81).

## Claims

1. System for sterilizing medical instruments (1), comprising:
- a testing station (20-26) for checking delivered instruments;
- an instrument storage area (40) for temporary storage of tested instruments;
- a packing station (70) for equipping instrument carriers (2) with instruments from the instrument storage area;
- at least one robot (30) for transporting instruments from the testing station to the instrument storage area and/or for equipping instrument carriers at the packing station with instruments from the instrument storage area, in particular on the basis of an operation-specific predetermined packing pattern; and
- a sterilizing apparatus (91, 92) for sterilizing instrument carriers equipped with instruments from the packing station,
**characterized in that** the instrument storage area comprises:
- an input region (41) and, in particular at a distance therefrom, an output region (42) for the selective output of different instruments;
- a storage system for placing an instrument at different storage locations; and
- an instrument recognition means (43) for detecting instruments in the output region, in particular for picking up instruments with the at least one robot and/or examining the instruments.

2. System according to Claim 1, **characterized in that** the testing station comprises:
- a manual workstation (20-23) for checking instruments, in particular for servicing and/or disposing of them, by at least one operator; and/or
- a pick-up station (24) for picking up instruments with the at least one robot.

3. System according to the preceding claim, **characterized in that**
- the testing station comprises:
- a passive or actuated transport device (25) for transporting instruments from the manual workstation to the pick-up station; and/or
- an instrument recognition means (26) for detecting instruments at the pick-up station, in particular for picking up instruments with the at least one robot and/or examining the instruments; and/or
- the manual workstation comprises
- a cleaning station (22) for manually cleaning instruments;
- a repair station (20) for manually repairing instruments; and/or
- a disposal station (23) for manually disposing of instruments.

4. System according to any of the preceding claims, **characterized in that** the packing station comprises:
- an instrument recognition means (71) for detecting instruments and/or instrument carriers at the packing station, in particular for equipping instrument carriers with instruments using the at least one robot and/or examining the instrument carriers and/or instruments.

5. System according to any of the preceding claims, **characterized in that** the instrument recognition means (26) for detecting instruments at the pick-up station, the instrument recognition means (43) for detecting instruments in the output region and/or the instrument recognition means (71) for detecting instruments at the packing station comprise at least one transceiver and/or at least one camera, in particular image processing means, for ascertaining the pose and/or identity of the instruments.

6. System according to any of the preceding claims, **characterized by**:
- a cleaning and/or disinfection station (10) for cleaning and/or disinfecting instruments and a passive or actuated delivery device (11) for delivering these instruments from the cleaning and/or disinfection station to the testing station; and/or by:
- an instrument-carrier-with-instrument storage area (110) for storing instrument carriers equipped with instruments from the sterilizing apparatus;
- an instrument-carrier-without-instrument storage area (60) for storing empty instrument carriers; and/or
- a transport system (50; 80) for the removal of empty instrument carriers from the cleaning and/or disinfection station and/or the testing station and/or the instrument-carrier-without-instrument storage area and/or to the instrument-carrier-without-instrument storage area and/or the packing station and/or for the removal of instrument carriers equipped with instruments from the packing station and/or the sterilization apparatus and/or the instrument-carrier-with-instrument storage area and/or to the sterilizing apparatus and/or the instrument-carrier-with-instrument storage area, in particular by means of driverless transport vehicles (81).

7. Method for sterilizing medical instruments (1) by means of a system according to any of the preceding claims, including the steps of:
- checking delivered instruments at a or the testing station (20-26);
- temporarily storing checked instruments using an or the instrument storage area (40);
- sterilizing instrument carriers (2) equipped with instruments from an or the packing station at a or the sterilization apparatus (91, 92);
and at least one of the steps of:
- transporting instruments from the testing station to the instrument storage area and/or equipping instrument carriers at the packing station with instruments from the instrument storage area using at least one or the at least one robot (30), in particular on the basis of an operation-specific predetermined packing pattern.

8. Method according to the preceding claim, **characterized by** at least one of the steps of:
- checking instruments, in particular servicing and/or disposing of them, by at least one operator (B) at a or the manual workstation (20-23); and/or
- picking up instruments with the at least one robot at a or the pick-up station (24) of the testing station.

9. Method according to the preceding claim, **characterized by** at least one of the steps of:
- passive or actuated transport of instruments from the manual workstation to the pick-up station of the testing station with a or the transport device (25);
- detecting instruments at the pick-up station with an or the instrument recognition means (26), in particular picking up instruments with the at least one robot and/or examining the instruments on the basis of the instrument recognition means;
- manually cleaning instruments at a or the cleaning station (22);
- manually repairing instruments at a or the repair station (20); and/or
- manually disposing of instruments at a or the disposal station (23).

10. Method according to any of the preceding Claims 7 to 9, **characterized by** at least one of the steps of:
- handing over instruments with the at least one robot at an or the input region (41) of the instrument storage area;
- selectively handing out different instruments to a or the output region (42) of the instrument storage area;
- placing at least one instrument at one of several different storage locations by a or the storage system based on a storage management means; and/or
- detecting instruments at an or the output region (42) with an or the instrument recognition means (43), in particular picking up instruments with the at least one robot and/or examining the instruments on the basis of the instrument recognition means.

11. Method according to any of the preceding Claims 7 to 10, **characterized by** the step of:
- detecting instruments and/or instrument carriers at the packing station, in particular equipping instrument carriers with instruments using the at least one robot and/or examining the instrument carriers and/or instruments, on the basis of an or the instrument recognition means (71).

12. Method according to any of the preceding Claims 7 to 11, **characterized by** at least one of the steps of:
- cleaning and/or disinfecting instruments at a or the cleaning and/or disinfection station (10) and passive or actuated delivery of these instruments from the cleaning and/or disinfection station to the testing station with a or the delivery device (11);
- storing instrument carriers equipped with instruments from the sterilizing apparatus in an or the instrument-carrier-with-instrument storage area (110);
- storing empty instrument carriers in an or the instrument-carrier-without-instrument storage area (60); and/or
- removing empty instrument carriers from a or the cleaning and/or disinfection station (10) and/or the testing station and/or an or the instrument-carrier-without-instrument storage area (60) and/or to an or the instrument-carrier-without-instrument storage area (60) and/or the packing station and/or removing instrument carriers equipped with instruments from the packing station and/or the sterilization apparatus and/or the instrument-carrier-with-instrument storage area and/or to the sterilizing apparatus and/or the instrument-carrier-with-instrument storage area with a or the transport system (50; 80), in particular by means of its driverless transport vehicles (81).

## Revendications

1. Système pour stériliser des instruments médicaux (1), qui présente :
- une station de test (20-26) pour tester des instruments livrés ;
- un magasin d'instruments (40) pour stocker temporairement des instruments testés ;
- une station d'emballage (70) pour équiper des supports d'instruments (2) avec des instruments provenant du magasin d'instruments ;
- au moins un robot (30) pour transporter des instruments de la station de test au magasin d'instruments et/ou pour équiper des supports d'instruments au niveau de la station d'emballage avec des instruments provenant du magasin d'instruments, notamment sur la base d'un modèle d'emballage prédéfini spécifique à l'opération ; et
- un dispositif de stérilisation (91, 92) pour stériliser des supports d'instruments équipés d'instruments provenant de la station d'emballage, **caractérisé en ce que** le magasin d'instruments présente :
- une zone d'entrée (41) et une zone de sortie (42), notamment espacée de celle-ci, pour la sortie sélective de différents instruments ;
- un système de stockage pour déposer un instrument à différents emplacements de stockage ; et
- une reconnaissance d'instruments (43) pour détecter des instruments au niveau de la zone de sortie, notamment pour prélever des instruments avec l'au moins un robot et/ou contrôler les instruments.

2. Système selon la revendication 1, **caractérisé en ce que** la station de test présente :
- un poste de travail manuel (20-23) pour le test, notamment l'entretien et/ou l'élimination, d'instruments par au moins un opérateur ; et/ou
- une station de prélèvement (24) pour prélever des instruments avec l'au moins un robot.

3. Système selon la revendication précédente, **caractérisé en ce que**
- la station de test présente :
- un appareil de transport passif ou actionné (25) pour transporter des instruments depuis le poste de travail manuel jusqu'à la station de prélèvement ; et/ou
- une reconnaissance d'instruments (26) pour détecter des instruments au niveau de la station de prélèvement, notamment pour prélever des instruments avec l'au moins un robot et/ou contrôler les instruments ; et/ou
- le poste de travail manuel présente
- une station de nettoyage (22) pour le nettoyage manuel d'instruments ;
- une station de réparation (20) pour la réparation manuelle d'instruments ; et/ou
- une station d'élimination (23) pour l'élimination manuelle d'instruments.

4. Système selon l'une quelconque des revendications précédentes, **caractérisé en ce que** la station d'emballage présente :
- une reconnaissance d'instruments (71) pour détecter des instruments et/ou des supports d'instruments au niveau de la station d'emballage, notamment pour équiper des supports d'instruments avec des instruments avec l'au moins un robot et/ou contrôler les supports d'instruments et/ou les instruments.

5. Système selon l'une quelconque des revendications précédentes, **caractérisé en ce que** la reconnaissance d'instruments (26) pour détecter des instruments au niveau de la station de réception, la reconnaissance d'instruments (43) pour détecter des instruments au niveau de la zone de sortie et/ou la reconnaissance d'instruments (71) pour détecter des instruments au niveau de la station d'emballage présente au moins un dispositif d'émission-réception et/ou au moins une caméra, notamment un traitement d'images, pour déterminer une position et/ou une identité des instruments.

6. Système selon l'une quelconque des revendications précédentes, **caractérisé par** :
- une station de nettoyage et/ou de désinfection (10) pour nettoyer et/ou désinfecter des instruments et un appareil de livraison passif ou actionné (11) pour livrer ces instruments de la station de nettoyage et/ou de désinfection à la station de test ; et/ou par :
- un magasin de supports d'instruments avec instruments (110) pour stocker des supports d'instruments équipés d'instruments provenant du dispositif de stérilisation ;
- un magasin de supports d'instruments sans instruments (60) pour stocker des supports d'instruments vides ; et/ou
- un système de transport (50 ; 80) pour évacuer des supports d'instruments vides de la station de nettoyage et/ou de désinfection et/ou de la station de test et/ou du magasin de supports d'instruments sans instruments et/ou vers le magasin de supports d'instruments sans instruments et/ou vers la station d'emballage et/ou pour évacuer des supports d'instruments équipés d'instruments de la station d'emballage et/ou du dispositif de stérilisation et/ou du magasin de supports d'instruments avec instruments et/ou vers le dispositif de stérilisation et/ou le magasin de supports d'instruments avec instruments, notamment à l'aide de véhicules de transport sans conducteur (81).

7. Procédé de stérilisation d'instruments médicaux (1) au moyen d'un système selon l'une quelconque des revendications précédentes, avec les étapes suivantes :
- le test d'instruments livrés au niveau d'une ou de la station de test (20-26) ;
- le stockage intermédiaire d'instruments testés au moyen d'un ou du magasin d'instruments (40) ;
- la stérilisation de supports d'instruments (2) équipés d'instruments provenant d'une ou de la station d'emballage au niveau d'un ou du dispositif de stérilisation (91, 92) ;
et au moins une des étapes suivantes :
- le transport d'instruments depuis la station de test jusqu'au magasin d'instruments et/ou l'équipement de supports d'instruments au niveau de la station d'emballage avec des instruments provenant du magasin d'instruments avec au moins un ou l'au moins un robot (30), notamment sur la base d'un modèle d'emballage prédéfini spécifique à l'opération.

8. Procédé selon la revendication précédente, **caractérisé par** au moins une des étapes suivantes :
- le test, notamment l'entretien et/ou l'élimination, d'instruments par au moins un opérateur (B) au niveau d'un ou du poste de travail manuel (20-23) ; et/ou
- le prélèvement d'instruments avec l'au moins un robot au niveau d'une ou de la station de prélèvement (24) de la station de test.

9. Procédé selon la revendication précédente, **caractérisé par** au moins une des étapes suivantes :
- le transport passif ou actionné d'instruments depuis le poste de travail manuel jusqu'à la station de prélèvement de la station de test avec un ou l'appareil de transport (25) ;
- la détection d'instruments au niveau de la station de prélèvement avec une ou la reconnaissance d'instruments (26), notamment le prélèvement d'instruments avec l'au moins un robot et/ou le contrôle des instruments sur la base de la reconnaissance d'instruments ;
- le nettoyage manuel d'instruments au niveau d'une ou de la station de nettoyage (22) ;
- la réparation manuelle d'instruments au niveau d'une ou de la station de réparation (20) ; et/ou
- l'élimination manuelle d'instruments au niveau d'une ou de la station d'élimination (23).

10. Procédé selon l'une quelconque des revendications précédentes 7 à 9, **caractérisé par** au moins une des étapes suivantes :
- le déchargement d'instruments avec l'au moins un robot au niveau d'une ou de la zone d'entrée (41) du magasin d'instruments ;
- la sortie sélective de différents instruments au niveau d'une ou de la zone de sortie (42) du magasin d'instruments ;
- le dépôt d'au moins un instrument au niveau d'un de plusieurs emplacements de stockage différents par un ou le système de stockage sur la base d'une gestion de stock ; et/ou
- la détection d'instruments au niveau d'une ou de la zone de sortie (42) avec une ou la reconnaissance d'instruments (43), notamment le prélèvement d'instruments avec l'au moins un robot et/ou le contrôle des instruments sur la base de la reconnaissance d'instruments.

11. Procédé selon l'une quelconque des revendications précédentes 7 à 10, **caractérisé par** l'étape suivante :
- la détection d'instruments et/ou de supports d'instruments au niveau de la station d'emballage, notamment l'équipement de supports d'instruments avec des instruments avec l'au moins un robot et/ou le contrôle des supports d'instruments et/ou des instruments, sur la base d'une ou de la reconnaissance d'instruments (71).

12. Procédé selon l'une quelconque des revendications précédentes 7 à 11, **caractérisé par** au moins une des étapes suivantes :
- le nettoyage et/ou la désinfection d'instruments au niveau d'une ou de la station de nettoyage et/ou de désinfection (10) et la livraison passive ou actionnée de ces instruments de la station de nettoyage et/ou de désinfection à la station de test avec un ou l'appareil de livraison (11) ;
- le stockage de supports d'instruments équipés d'instruments provenant du dispositif de stérilisation dans un ou le magasin de supports d'instruments équipés d'instruments (110) ;
- le stockage de supports d'instruments vides dans un ou le magasin de supports d'instruments sans instruments (60) ; et/ou
- l'évacuation de supports d'instruments vides d'une ou de la station de nettoyage et/ou de désinfection (10) et/ou de la station de test et/ou d'un ou du magasin de supports d'instruments sans instruments (60) et/ou vers un ou le magasin de supports d'instruments sans instruments (60) et/ou la station d'emballage et/ou l'évacuation de supports d'instruments équipés d'instruments de la station d'emballage et/ou du dispositif de stérilisation et/ou du magasin de supports d'instruments avec instruments et/ou vers le dispositif de stérilisation et/ou le magasin de supports d'instruments avec instruments avec un ou le système de transport (50 ; 80), notamment à l'aide de ses véhicules de transport sans conducteur (81).
